# EUROPEAN PATENT APPLICATION

(11) **EP 4 517 777 A1**
(43) Date of publication of application: **05.03.2025**
(21) Application number: 23193826.7
(22) Date of filing: 29.08.2023
(51) Int. Cl.: G16H 50/70, G16H 50/20, G16H 50/30, A61B 5/00

(54) **METHOD FOR TRAINING A SLEEP-RELATED EVENT CLASSIFICATION MODEL**

(71) Applicant: Koninklijke Philips N.V., 5656 AG Eindhoven (NL)
(72) Inventor: VAN DER AAR, Jaap, Eindhoven (NL); VAN DEN ENDE, Daan Anton, Eindhoven (NL); DOS SANTOS DA FONSECA, Pedro Miguel Ferreira, 5656AG Eindhoven (NL); VAN GILST, Merel Marietje, Eindhoven (NL); OVEREEM, Sebastiaan, Eindhoven (NL); PERI, Elisabetta, Eindhoven (NL)
(74) Representative: Philips Intellectual Property & Standards

(57) **Abstract**

Disclosed is a method of training a sleep-related event classification model. The method may comprise a step of providing a pre-trained classification model. The classification model may be configured to classify one or more sleep-related events of a subject based on one or more physiological measurements of the subject. The classification model may be pre-trained using first training data associated with a source population of subjects. The method may comprise a step of finetuning the classification model using second training data associated with a target population of subjects. In addition, a corresponding method of classifying one or more sleep-related events of a subject as well as a corresponding computer program, data processing apparatus or system and data structure is provided.

## Description

### TECHNICAL FIELD

The present disclosure generally relates to the field of sleep analysis, and more particularly to techniques for training a sleep-related event classification model as well as classifying one or more sleep-related events of a subject using the trained sleep-related event classification model. Certain embodiments may provide for target population-based finetuning of the sleep-related event classification model, thereby overcoming data quality issues (e.g., scarcity) resulting in improved classification performance.

### BACKGROUND

In recent years, there has been a push towards more automated classification of sleep-related events (e.g., sleep staging). Especially the use of deep learning methods for automated sleep staging has yielded promising results, showing similar or superior performance as in manual sleep classification. However, to reach expert-level performance using deep learning models, hundreds of sleep recordings are required for training, a quantity that is often unavailable in sleep studies. Although a possible solution to data scarcity is to pretrain a model on large, publicly available datasets (the source domain) and afterwards apply it to the smaller dataset of interest (the target domain), there are potentially mismatches between the datasets, caused by changes in the characteristics of the population and/or sensor location.

Recently, transfer learning has been proposed as solution for dealing with data scarcity and data mismatches. Transfer learning is a machine learning technique that has been applied in other domains previously (e.g., image processing) and recently has been applied in some studies for automated sleep staging where transfer learning was used to tune a sleep staging model to a new electrode location (e.g., from frontal EEG electrode to behind-the-ear EEG electrode) or to tune to characteristics of a specific subject (by training the model on part of the data of the subject).

However, the known approaches fail to provide a method which is generally applicable to the classification task of sleep-related events, because they provide solutions which are merely applicable to very specific use cases (e.g., change of electrode location or tuned to a specific subject).

It is therefore an objective of the present disclosure to provide for a generally applicable method for training sleep-related event classification models and for overcoming data quality issues (e.g., scarcity) resulting in improved classification performance, thereby overcoming the above-mentioned disadvantages of the prior art at least in part.

### SUMMARY OF THE DISCLOSURE

The objective is solved by the subject-matter defined in the independent claims. Advantageous modifications of embodiments of the present disclosure are defined in the dependent claims as well as in the description and the figures.

As a general overview, certain aspects of the present disclosure provide for a target population-based finetuning of sleep-related event classification models.

One aspect of the present disclosure relates to a method of training a sleep-related event classification model. The method may comprise a step of providing a pre-trained classification model. The classification model may be configured to classify one or more sleep-related events of a subject based on one or more physiological measurements of the subject. The classification model may be pre-trained using first training data associated with a source population of subjects. The method may comprise a step of finetuning the classification model using second training data associated with a target population of subjects.

This way, a sleep-related event classification model can be finetuned to a specific target population, which combines the benefits of a pre-trained model (e.g., a general classification model trained on a larger data set of a source population, i.e., the first training data) with the benefits of a finetuned model (e.g., a specific classification model trained on a small, but highly specific data set of a target population, i.e., the second training data). As a result, the trained model is able to accurately perform automated classification tasks irrespective of data scarcity issues. This is because the finetuning typically requires less training data as compared to the pre-training.

Throughout the present disclosure, a sleep-related event may relate to any type of event related to a subject before (e.g., falling asleep), during (e.g., deep-sleep) and/or after (waking up) sleep. A sleep-related event may take place during a period where it is likely that a subject is trying (or somehow has the opportunity) to sleep. However, a sleep-related event may not require the subject being asleep (e.g., the subject usually will take some time to fall asleep, will wake up several times during the period or will try but fail to -quickly- fall asleep). All of these sleep-related events provide relevant information for the task of sleep-related event classification. A sleep-related event may be associated with a sleep stage. Further examples of sleep-related events will be explained throughout the present disclosure. The first training data associated with a source population of subjects may be different from the second training data associated with a target population of subjects. A target population as referred to throughout the present disclosure may relate to a population of at least two subjects. Generally speaking, the term "population" may be understood as referring to a group of individuals, rather than a single individual. According to another aspect of the present disclosure, the method may further comprise a step of determining an actual or expected insufficient performance of the pre-trained classification model for the subject. The step of finetuning the classification model may be performed in response to the step of determining the insufficient performance.

This way, if it is determined that the pre-trained model does not (i.e., actually) or will not (i.e., expectedly) perform sufficiently well, finetuning the model can be implemented as a follow up step so that the performance of the model can be improved to a point where it performs sufficiently well.

According to another aspect of the present disclosure, the step of determining the insufficient performance of the pre-trained classification model for the subject may comprise a step of determining that at least one characteristic of the subject mismatches the source population of subjects.

This way, if at least one characteristic of the subject mismatches the source population of subjects, it can be assumed that the pre-trained model cannot or will not perform sufficiently well due to the differences in data. Accordingly, whether finetuning is required can be efficiently determined.

According to another aspect of the present disclosure, the method may further comprise a step of selecting the second training data such that at least one characteristic of the subject which mismatches the source population of subjects matches the target population of subjects. The step of finetuning the classification model may be performed using the selected second training data. The method may comprise incrementally selecting portions of second training data and finetuning the classification model using the selected portion of second training data until a performance of the classification model is sufficient.

This way, the second training data is selected in a way that avoids mismatches between the subject and the target population. Accordingly, the model's performance is no longer negatively affected by mismatching samples. The incremental selection of portions and finetuning may avoid overfitting. Also, unnecessary training iterations can be avoided resulting in less computational resources required for the overall training.

According to another aspect of the present disclosure, the at least one characteristic of the subject may comprise one or more of: an age of the subject, a medical disorder of the subject, in particular a sleep-related medical disorder, such as insomnia, obstructive sleep apnea, or rapid eye movement sleep behavior disorder, a comorbid, non-sleep related, medical disorder, a body mass index of the subject, a medicine use of the subject, a therapy use of the subject, information about an environment of the subject or any combination thereof.

This way, characteristics are provided which alone or in combination can be the reason for adversely affecting the physiological measured signal of the subject. Therefore, determining mismatches based on one or more of these characteristics avoids the model being faced with inconsistent data.

According to another aspect of the present disclosure, the method may further comprise a step of applying the pre-trained classification model to one or more physiological measurements of the subject to determine an amount of second training data for the step of finetuning.

This way, an indication can be derived (e.g., by using cross validation between the first and the second training data) about how much more additional data from subjects with similar characteristics is needed for reliable and sufficient performance.

Another aspect of the present disclosure relates to a method of classifying one or more sleep-related events of a subject. The method may comprise a step of providing a trained classification model. The trained classification model may be trained using a method of training a sleep-related event classification model according to any one of the aspects described herein. The method may comprise a step of providing one or more physiological measurements of the subject. The method may comprise a step of classifying one or more sleep-related events of the subject based at least in part on the one or more physiological measurements using the classification model to generate a classification result. The method may comprise a step of providing the classification result.

By utilizing the trained classification model, which has been trained using the training method according to aspects of the present disclosure, the step of classifying one or more sleep-related events of the subjects will yield improved classification results compared to the methods known in the art.

Throughout the present disclosure, providing physiological measurements may be done by utilizing corresponding sensors (e.g., EEG, EMG, EOG etc.) for measuring and corresponding communication means (e.g., database transfers/requests, wired or wireless communication protocols etc.) for accessing and serving of the data. Typically, providing the classification result is done via displaying the result on a display or any other corresponding mean for displaying (e.g., smartphone, tablet, computer display or the like) or via transferring the result to a follow up application utilizing the result for further technical processing.

According to another aspect of the present disclosure, the classification model may comprise a machine-learning model, more particularly a deep learning model, more particularly at least one neural network, more particularly at least one convolutional neural network, more particularly DeepSleepNet or TinySleepNet.

It shows that the training method of the present disclosure can be applied to a wide range of models and thus provides a generally applicable method for training sleep-related classification models of all types.

According to another aspect of the present disclosure, the one or more physiological measurements may include one or more of: polysomnography data, electroencephalography data, electromyography data, photoplethysmography data, electrocardiography data, electrooculography data, actigraphy data, heart rate variability data, movement pattern data, breathing pattern data, peripheral oxygen saturation data, or any combination thereof.

This way, data is provided which best describes sleep-related events. Accordingly, a model trained on one or more of these data categories can achieve a sufficient performance on the classification task.

According to another aspect of the present disclosure, the classification model may be configured to classify a sleep-related event of the subject into one or more of: a waking stage, a sleep stage, such as a non-rapid eye movement sleep stage or a rapid eye movement sleep stage, a cortical event, such as an arousal, sleep spindle, a K-complex, a slow wave, a non-cortical event, such as an obstructive or central apnea, a hypopnea, a respiratory effort related arousal, or any combination thereof. The non-rapid eye movement sleep stage may comprise a stage 1 non-rapid eye movement sleep stage, a stage 2 non-rapid eye movement sleep stage or a stage 3 non-rapid eye movement sleep stage.

It shows that the training method of the present disclosure may be applied to a wide range of sleep-related events and is not limited to the classification of a specific use case.

According to another aspect of the present disclosure, the first training data associated with the source population may map one or more physiological measurements of subjects of the source population onto a plurality of sleep stages, in particular the plurality of sleep stages as described herein. This way, a more finely granular model is trained which not only classifies sleep-related events, but the very specific sleep stage of a subject.

According to another aspect of the present disclosure, the sleep-related event classification model may have been trained using supervised or semi-supervised learning.

This way, depending on the amount of available labeled data samples either supervised (e.g., if sufficient samples are available) or semi-supervised (e.g., if less samples than required are available) can be applied to still obtain a sufficiently well performing classification model.

Another aspect of the present disclosure relates to a computer program, or a computer-readable medium storing a computer program. The computer program may comprise instructions which, when the program is executed on a computer and/or a computer network, may cause the computer and/or the computer network to carry out the method(s) of any one of the aspects described herein.

Another aspect of the present disclosure relates to a data processing apparatus or system comprising means for carrying out the method(s) of any one of the aspects described herein.

Another aspect of the present disclosure relates to a data structure comprising a trained sleep-related event classification model. The model may be trained using the method of any one of the aspects described herein.

### BRIEF DESCRIPTION OF THE DRAWINGS

The disclosure may be better understood by reference to the following drawings:
Fig. 1 An overview of a method for training a sleep-related event classification model in accordance with embodiments of the present disclosure.
Fig. 2 An overview of a method of classifying one or more sleep-related events of a subject in accordance with embodiments of the present disclosure.
Fig.3 A first comparison of performance results of the sleep-related even classification model in accordance with embodiments of the present disclosure.
Fig.4 A second comparison of performance results of the sleep-related even classification model in accordance with embodiments of the present disclosure.
Fig. 5 A third comparison of performance results of the sleep-related even classification model in accordance with embodiments of the present disclosure.
Fig. 6 An exemplary implementation of a data-processing system in accordance with embodiments of the present disclosure.

### DETAILED DESCRIPTION

In the following, representative embodiments illustrated in the accompanying drawings will be explained. It should be understood that the illustrated embodiments and the following descriptions refer to examples which are not intended to limit the embodiments to one preferred embodiment.

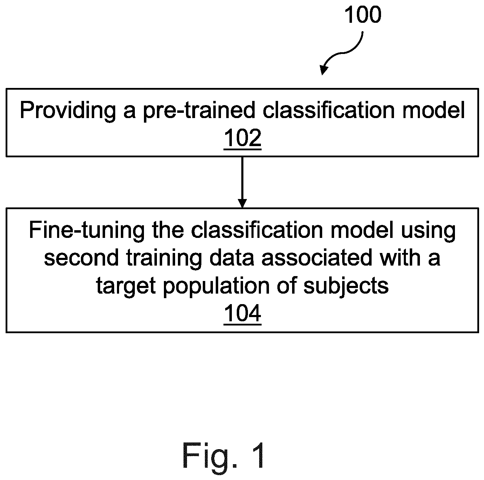

Fig. 1 illustrates an overview of a method 100 for training a sleep-related event classification model in accordance with an exemplary embodiment. The method 100 may comprise a step 102 of providing a pre-trained classification model. The classification model may be configured to classify one or more sleep-related events of a subject based on one or more physiological measurements of the subject. The classification model may be pre-trained using first training data associated with a source population of subjects. The method 100 may comprise a step 104 of finetuning the classification model using second training data associated with a target population of subjects.

The method 100 may further comprise a step of determining an actual or expected insufficient performance of the pre-trained classification model for the subject. The step 104 of finetuning the classification model may be performed in response to the step of determining the insufficient performance.

The step of determining the insufficient performance of the pre-trained classification model for the subject may comprise a step of determining that at least one characteristic of the subject mismatches the source population of subjects.

The method 100 may further comprise a step of selecting the second training data such that at least one characteristic of the subject which mismatches the source population of subjects matches the target population of subjects. The step 104 of finetuning the classification model may be performed using the selected second training data. The method may comprise incrementally selecting portions of second training data and finetuning the classification model using the selected portion of second training data until a performance of the classification model is sufficient.

The at least one characteristic of the subject may comprise one or more of: an age of the subject, a medical disorder of the subject, in particular a sleep-related medical disorder, such as insomnia, obstructive sleep apnea, or rapid eye movement sleep behavior disorder, a comorbid, non-sleep related, medical disorder, a body mass index of the subject, a medicine use of the subject, a therapy use of the subject or any combination thereof. Another characteristic may be a gender of the subject, which may be of relevance when it comes to cardiovascular related disorder or cardiovascular based sleep staging. Another characteristic may be information about an environment of the subject. The information about the environment of the subject as referred to throughout the present disclosure may comprise information about the environment in which the data was acquired (e.g., weather conditions, such as hot, cold, humid or dry, a noise level, lighting conditions etc.), time information (e.g., a time of the year, such as summer or winter, etc.), and/or geographical information (e.g., a location in which the data was acquired, etc.). For example, weather conditions such as temperature and humidity may cause artefacts (e.g., electrodermal artefacts such as sweating) which may disturb the measurement signal (e.g., the EEG signal). For example, geographical information such as the location may also affect the model, because habits of the subjects (e.g., with respect to sleep hygiene, alcohol intake, smoking etc.) may differ based on whether the subject was measured in a sleep lab setting or at home.

For example, if a comorbid, non-sleep related, medical disorder such as a cardiac condition is present, the corresponding arrythmias can lead to degradation of performance if the model is not adapted (i.e., finetuned) accordingly. In another example, if the presence of congestive heart failure may increase the incidence and proportion of central sleep apneas in relation to other sleep disordered breathing events. The therapy used by a subject (e.g., the use of positive airway pressure, PAP, therapy to treat OSA) will naturally reduce the number of residual sleep-disordered breathing events. In this case it might be advantageous to adapt the classification model to be more sensitive to a) less events, and b) of a "milder" type - i.e., shorter duration, partial instead of complete obstructions, etc. In another example, the use of PAP specifically may trigger therapy emergent central sleep apnea. A more general classifier (e.g., a pre-trained model) might underestimate the presence of this type of sleep disordered breathing events, because they are naturally less occurring, e.g., versus obstructive apneas, and especially hypopneas. When using PAP, the model could be finetuned to be more sensitive to the detection of central apneas, since this has to be flagged so the patient can be switched to other therapy modalities, e.g., ASV.

A population (e.g., a source and/or target population) as referred to throughout the present disclosure may be defined based on one or more criteria (e.g., a minimum number of subjects, a measure of fit, a spread of characteristics of the subjects etc.). For example, a population may have to include at least 100 subjects in one particular embodiment. Whether the number of subjects is sufficient may be determined by measuring the fit of the model (e.g., the classification performance of the model being trained on the population). As another example, the spread of characteristics of the population (i.e., heterogeneity) can be determined based on age, medical disorder(s) or sleep disorder(s) (e.g., mild disorder, moderate disorder etc.).

A number of subjects needed for finetuning the classification model (i.e., subjects of the target population) may be determined based on a number of subjects used for the pre-training (i.e., subjects of the source population). For example, a fraction value, such as 5% or 10%, for instance, may be used for determining the number of subjects. In an example, in which 100 subjects (of the source population) were used for pre-training, 5 or 10 subjects (of the target population) may be needed for finetuning the classification model.

It is to be understood that the present method 100 for training a classification model of sleep-related events can be applied to any type of sleep-related event such as sleep stages, other cortical events such as arousals, spindles, K-complexes, slow waves, or non-cortical (but still sleep-related) events such as obstructive or central apneas, hypopneas, or respiratory effort related arousals (RERAs). These are all (useful) classification tasks during the period where a subject may be asleep and are all based on one or more (albeit not necessarily all the same) physiological measurements. Environmental characteristics, such as the information about an environment of the subject(s), may also play a role as they can affect how sleep-related events manifest themselves in the data set(s). Other event related characteristics such as duration of the event may also play a role. For example, sleep-related events of short duration within the target population may be of importance if the source population does not comprise such sleep-related events of short duration (e.g., due to low sampling rate, different applied filters etc.). Adaptation (i.e., finetuning) to a target population is relevant for all of these as adapting to the different physiological/medical, environmental and/or event related characteristics of the population is necessary to achieve sufficient performance.

For example, presence and intensity of sleep spindles (traditionally visible in EEG primarily during N2 sleep) can be altered by different types of medication. Adapting the model for specific medication-intake (taken by a given population), could allow the model to account for these differences. The same applies to the use case of detection of slow waves. Although they are still present in older subjects, they have a greatly diminished amplitude. Allowing the model to adapt to an older population for this task would likely improve its performance in the detection of these events. The same applies to the use case of respiratory events, for example, when using CPAP, some subjects develop central apnea during therapy. A general algorithm (e.g., a pre-trained model) that detects sleep disordered beathing events of any type, could probably be improved to increase the sensitivity to central apneas, a crucial aspect of detecting this pathology (these patients actually have to be moved to different PAP therapy modalities). These non-limiting examples are merely used to illustrate potential practical use cases of how the aspects of the present disclosure can be generally used for different sleep-related classification tasks.

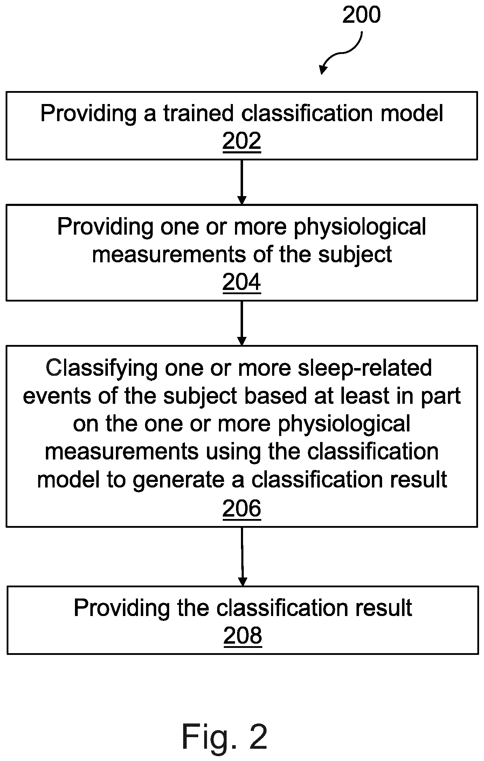

Fig. 2 illustrates an overview of a method 200 of classifying one or more sleep-related events of a subject in accordance with an exemplary embodiment. The method 200 may comprise a step 202 of providing a trained classification model. The trained classification model may be trained using the method 100 of training a sleep-related event classification model as described with respect to Fig. 1. The method 200 may comprise a step 204 of providing one or more physiological measurements of the subject. The method 200 may comprise a step 206 of classifying one or more sleep-related events of the subject based at least in part on the one or more physiological measurements using the classification model to generate a classification result. The method 200 may comprise a step 208 of providing the classification result.

The classification model as described with respect to Fig. 1 and/or Fig 2 may comprise a machine-learning model, more particularly a deep learning model, more particularly at least one neural network, more particularly at least one convolutional neural network, more particularly DeepSleepNet or TinySleepNet.

The one or more physiological measurements as described with respect to Fig.1 and/or Fig. 2 may include one or more of: polysomnography data, electroencephalography data, electromyography data, photoplethysmography data, electrocardiography data, electrooculography data, actigraphy data, heart rate variability data, movement pattern data, breathing pattern data, peripheral oxygen saturation data or any combination thereof.

The classification model as described with respect to Fig. 1 and/or Fig. 2 may be configured to classify a sleep-related event of the subject into one or more of: a waking stage, a sleep stage, such as a non-rapid eye movement sleep stage or a rapid eye movement sleep stage, a cortical event, such as an arousal, sleep spindle, a K-complex, a slow wave, a non-cortical event, such as an obstructive or central apnea, a hypopnea, a respiratory effort related arousal, or any combination thereof. The non-rapid eye movement sleep stage may comprise a stage 1 non-rapid eye movement sleep stage, a stage 2 non-rapid eye movement sleep stage or a stage 3 non-rapid eye movement sleep stage. The first training data as described with respect to Fig. 1 associated with the source population may map one or more physiological measurements of subjects of the source population onto a plurality of sleep stages, in particular the plurality of sleep stages as described above. The sleep-related event classification model may have been trained as described with respect to Fig. 1 using supervised or semi-supervised learning. For example, the classification may be configured to classify the sleep-related event as either a waking stage (e.g., the subject is awake) versus any other of the sleep-related events or sleep stages.

Mismatches between the general (e.g., pre-trained) model and target data (e.g., data from subjects of the target population) may be labeled. For example, a subject may be measured. Based on the measurements a mismatch may be identified (e.g., using outlier detection) and a label for the mismatch may be given (e.g., sleep disorder, age, BMI, medicine use etc.). This way a situation can be avoided in which physiological measured signals are (potentially) affected.

An indication of expected increased performance of the finetuned model can be obtained. For example, if both, the pre-trained model, and the finetuned model show certain agreement levels with respect to sleep-related events (e.g., sleep staging) on the (available) data. This may be determined using cross-validation. In addition, an indication of the reliability of the finetuned model may be derived based on the agreement levels combined with a derived variance of the (available) data. In case, the indicated performance and/or reliability is not sufficient, an indication of how much more additional data from subjects with similar characteristics is needed may be given to achieve a reliable and sufficient performance. Such an indication may be derived from incremental increases in performance of the finetuned model when step-by-step more data of similar characteristics is added.

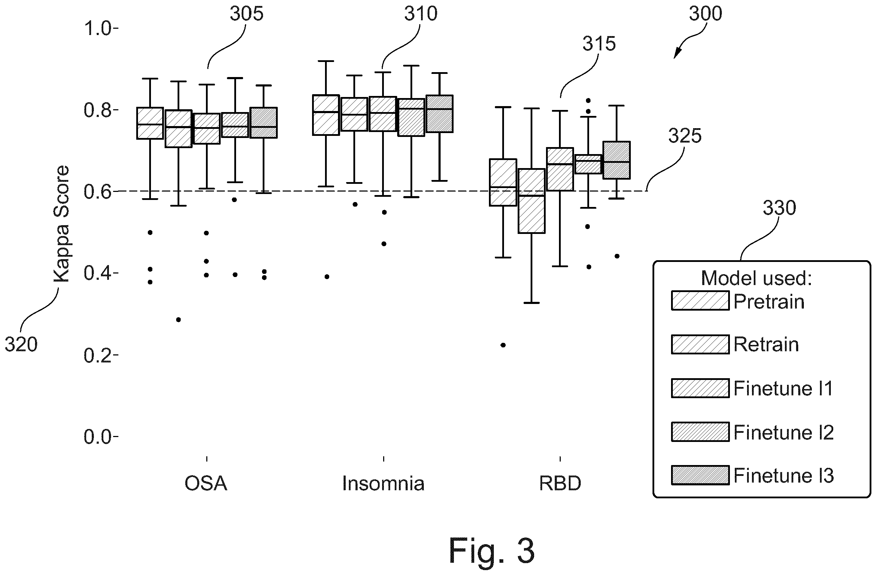

Fig. 3 illustrates a first comparison 300 of performance results of the sleep-related even classification model in accordance with embodiments of the present disclosure. In the example illustrated, the classification model comprises the TinySleepNet which was trained using the method 100. Overall, the first comparison 300 illustrates the improved performance of classification models trained using the method 100. In the illustrated example, the classified sleep-related events relate to sleep stages as described with respect to Figs. 1 and 2. It is to be understood that the application of the sleep-related even classification model according to the embodiments of the present disclosure is not limited to the examples shown in Fig. 3. Instead, this example is merely used to demonstrate the feasibility of the present invention. The method 100 for training is independent from the model and modality used. In this example, the physiological measurements of the subject include electroencephalography data which was captured using the F3-M2 electrode derivation. In this example, the electrode derivation was the same for both, the first and the second training data.

The first comparison 300 comprises performance results for three different sleep disorders, namely obstructive sleep apnea (OSA) 305, insomnia 310 and REM sleep behavioral disorder (RBD) 315. As evaluation metric for the illustrated performance results the Kappa Score 320 is used. The dotted line 325 relates to a Kappa Score of 0.6 which can be used as an indication for sufficient performance of the model. It is to be understood that other values indicating sufficient performance of the model are applicable.

As illustrated by the legend 330 differently trained classification models were used for evaluating the performance of the classification model according to the aspects of the present disclosure. The models used for the evaluation are a pretrained model, a retrained model as well as three models which were finetuned according to the aspects of the present disclosure.

The pretrained model relates to a pre-trained classification model which is pre-trained using first training data associated with a source population of subjects. In this example the pre-trained model was trained on a data set of (other) healthy sleep recordings.

The retrained model relates to a classification model which is trained using second training data associated with a target population of subjects. In this example, the retrained model was trained on a data set of available sleep recordings. In the context of the present disclosure, retraining may relate to training the model from scratch (i.e., the model is not pretrained).

The three finetuned models comprise a first finetuned model 11, a second finetuned model l2 and a third finetuned model l3. Each of the finetuned models relates to a classification model which is pre-trained using first training data associated with a source population of subjects and then finetuned by additional training on second training data associated with a target population of subjects. In this example, the finetuned models were pretrained on a data set of (other) healthy sleep recordings and then finetuned by additional training on available sleep recordings. Each of the three finetuned models was finetuned using a different amount n of second training data (e.g., number of subjects or available sleep recordings used for the finetuning). In the illustrated example, the amount of second training data used for the first finetuned model 11 was lower than the amount of second training data used for the second finetuned model l2 and the amount of second training data used for the second finetuned model l2 was lower than the amount of second training data used for the third finetuned model l3. The corresponding amount may be determined according to the aspects of the present disclosure.

Turning to the results of the OSA 305 classification and the insomnia 315 classification, the three finetuned models l1-l3 performed equally well or slightly better compared to the pretrain model and retrain model. For the OSA 305 classification the pretrained model achieved a sufficiently high Kapa Score (i.e., above 0.6). The same applies to the retrained model, because sufficient data samples (e.g., n=60) were available. In contrast, the finetuned models achieved a similar performance but require a lot less data (e.g., n=12 for l1, n=18 for l2, and n=30 for l3).

Turning to the results of the RBD 315 classification, the three finetuned models l1-l3 performed better compared to the pretrain model and the retrain model. This is because the data characteristics in comparison to the source population (i.e., the healthy recordings) have changed due to the disorder and/or age of the target population. RDB 315 relates to a rare sleep disorder in (mostly) elder patients. Accordingly, the respective data characteristics (e.g., age, the disorder itself, the medicine use etc.) as well as the resulting physiological measurements will be different from these the pre-trained model was trained on. As a result, the pretrained model underperformed (i.e., achieved a Kappa Score below 0.6). The same applies to the retrained model. The reason for this is data scarcity of the target population (e.g., n=22, due to the rarity of the disorder) available for the retraining resulting in a unrobust retrained model. In such a case, the advantages of the finetuned models according to the aspects of the present disclosure are clearly visible.

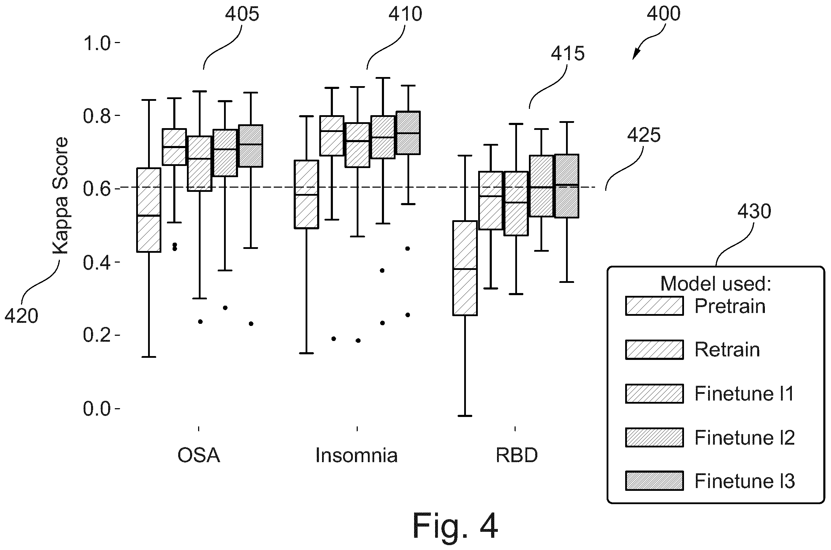

Fig. 4 illustrates a second comparison 400 of performance results of the sleep-related even classification model in accordance with embodiments of the present disclosure. In the example illustrated, the classification model comprises the TinySleepNet which was trained using the method 100. Overall, the second comparison 400 illustrates the improved performance of classification models trained using the method 100. In the illustrated example, the classified sleep-related events relate to sleep stages as described with respect to Figs. 1 and 2. It is to be understood that the application of the sleep-related even classification model according to the embodiments of the present disclosure is not limited to the examples shown in Fig. 4. Instead, this example is merely used to demonstrate the feasibility of the present invention. In this example, the physiological measurements of the subject include electroencephalography data. However, different than in the experiment underlying the results of Fig.3, a different electrode derivation was used for capturing of the second training data.

The second comparison 400 comprises performance results for three different sleep disorders, namely obstructive sleep apnea (OSA) 405, insomnia 410 and REM sleep behavioral disorder (RBD) 415. As evaluation metric for the illustrated performance results the Kappa Score 420 is used. The dotted line 425 relates to a Kappa Score of 0.6 which can be used as an indication for sufficient performance of the model. It is to be understood that other values indicating sufficient performance of the model are applicable.

As illustrated by the legend 430 differently trained classification models were used for evaluating the performance of the classification model according to the aspects of the present disclosure. The models used for the evaluation are a pretrained model, a retrained model as well as three models which were finetuned according to the aspects of the present disclosure.

The pretrained model relates to a pre-trained classification model which is pre-trained using first training data associated with a source population of subjects. In this example the pre-trained model was trained on a data set of (other) healthy sleep recordings.

The retrained model relates to a classification model which is trained using second training data associated with a target population of subjects. In this example, the retrained model was trained on a data set of available sleep recordings. In the context of the present disclosure, retraining may relate to training the model from scratch (i.e., the model is not pretrained).

The three finetuned models comprise a first finetuned model 11, a second finetuned model l2 and a third finetuned model l3. Each of the finetuned models relates to a classification model which is pre-trained using first training data associated with a source population of subjects and then finetuned by additional training on second training data associated with a target population of subjects. In this example, the finetuned models were pretrained on a data set of (other) healthy sleep recordings and then finetuned by additional training on available sleep recordings. Each of the three finetuned models was finetuned using a different amount n of second training data (e.g., number of subjects used for the finetuning). In the illustrated example, the amount of second training data used for the first finetuned model 11 was lower than the amount of second training data used for the second finetuned model l2 and the amount of second training data used for the second finetuned model l2 was lower than the amount of second training data used for the third finetuned model l3.

Turning to the results of the pretrained model it can be seen that the pretrained model underperformed in the OSA 405 classification, in the insomnia 410 classification as well as in the RBD 415 classification. The reason for this is that the characteristics of the first training data are different to the characteristics of the second training data, because a different electrode derivation was used.

If one compares the performance of the retrained model with the performance of the finetuned models, one can see that these models performed equally well in the OSA 405 classification and in the insomnia 410 classification. However, in the RBD 415 classification the retrained model underperformed (e.g., due to data scarcity). The same applies to the finetuned model 11, because the amount of the second data used for finetuning was not large enough. In contrast, the finetuned models l2 and l2 performed better and achieved a sufficiently high Kappa Score. This illustrates how the training method 100 of the present disclosure is able to not only improve classification performance but also determine the amount of data required for achieving a satisfying performance.

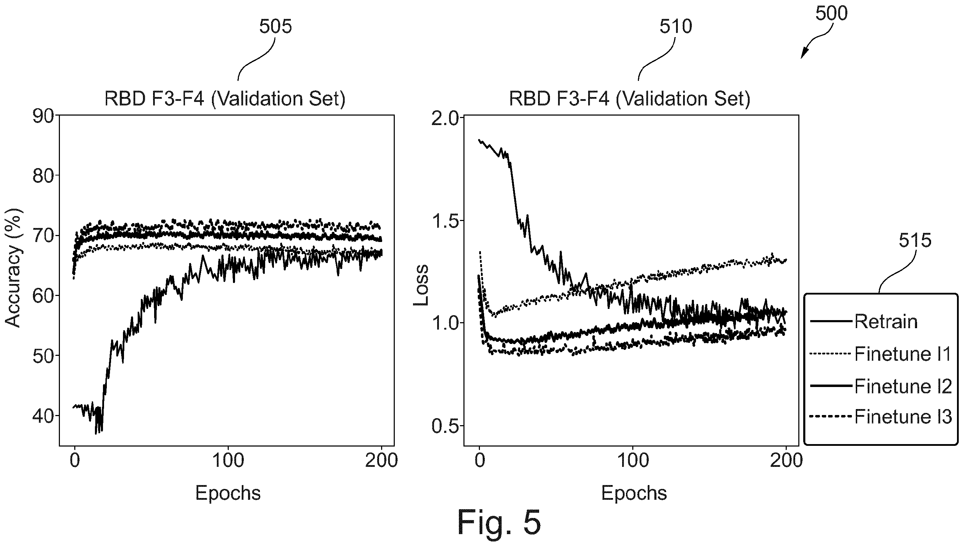

Fig. 5 illustrates a third comparison 500 of performance results of the sleep-related even classification model in accordance with embodiments of the present disclosure. The third comparison 500 illustrates the performance of the classification model with respect to classification accuracy 505 as well as with respect to loss 510.

As illustrated by the legend 515 differently trained classification models were used for evaluating the performance of the classification model according to the aspects of the present disclosure. The models used for the evaluation are a retrained model as well as three models which were finetuned according to the aspects of the present disclosure. The performance of each model was evaluated on a validation set (e.g., RBD group with F3-F4 derivation). These models may relate to these as described with respect to Fig. 3 and/or 4.

The retrained model relates to a classification model which is trained using second training data associated with a target population of subjects. In this example, the retrained model was trained on a data set of available sleep recordings. In the context of the present disclosure, retraining may relate to training the model from scratch (i.e., the model is not pretrained).

The three finetuned models comprise a first finetuned model 11, a second finetuned model l2 and a third finetuned model l3. Each of the finetuned models relates to a classification model which is pre-trained using first training data associated with a source population of subjects and then finetuned by additional training on second training data associated with a target population of subjects. In this example, the finetuned models were pretrained on a data set of (other) healthy sleep recordings and then finetuned by additional training on available sleep recordings. Each of the three finetuned models was finetuned using a different amount n of second training data (e.g., number of subjects used for the finetuning). In the illustrated example, the amount of second training data used for the first finetuned model 11 was lower than the amount of second training data used for the second finetuned model l2 and the amount of second training data used for the second finetuned model l2 was lower than the amount of second training data used for the third finetuned model l3.

As can be seen, accuracy 505 as well as loss 510 of the three finetuned models already achieves an optimal performance after less than 20 epochs. However, after the first 20 epochs the performance of the three finetuned models decreases and overfitting starts. The latter can be seen by the increase in the loss functions. In order to avoid the finetuned models from overfitting, early stoppage may be applied.

In contrast, the retrained model requires more than 10 times more epochs for achieving similar performance as the finetuned models after 20 epochs. This again illustrates the advantage of the present disclosure providing a way of achieving similar or better performance while requiring less computational resources due to the reduced number of required epochs.

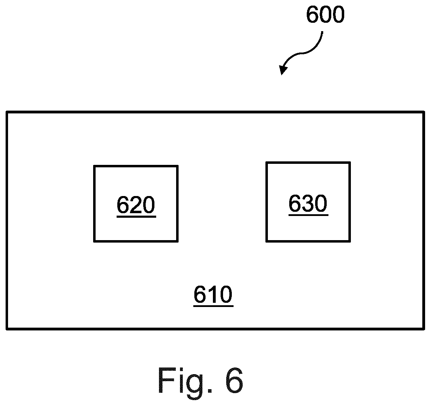

Fig 6 illustrates an exemplary implementation of a data-processing system 600 in accordance with embodiments of the present disclosure. The system 600 may comprise any suitable means for carrying out the method(s) according to the aspects of the present disclosure.

The method(s) of the present disclosure as for example explained with respect to Figs. 1-5, may be implemented as a software module 620 and integrated into an environment 610 in which the system 600 operates. In the environment 610 databases 630 may be stored. The environment 610 may for example be a sleep hospital, a company, a research platform etc.

A general automated model (e.g., a pre-trained model as referred to throughout the disclosure) is created to predict sleep stages based on (surrogate) sleep measures, such as, but not limited to, electroencephalography (EEG), electromyography (EMG), electrooculography (EOG), heart rate variability (HRV), movement and/or breathing patterns. This general model likely performs well in average subjects (e.g., a source population as referred to throughout the present disclosure) that represent their current database (e.g., one of the databases 630). However, assume, for example, a visit of a subject with REM sleep behavioral disorder (RBD), a rare sleep disorder in (mostly) elder patients. Physiological measures are likely distinct from most of the subjects in the current dataset, due to physiological characteristics related to e.g., the disorder itself, to the older age of the patient, and medicine use, causing underperformance of the automated model in that particular person and population.

Utilizing aspects of the present disclosure, the general model can quickly be tuned to the particular population (e.g., a target population as referred to throughout the present disclosure), by selecting all subjects from existing datasets (which can be e.g., publicly available or in-house data) with similar characteristics (e.g., a source population as referred to throughout the present disclosure) and following the finetuning procedure according to aspects of the present disclosure.

Feedback on the expected performance can be given by evaluating both the sleep stage performance of the general and tuned model through cross-validation, as well as an indication of the reliability (e.g., variance within the tuned model, and how often a "sufficient" performance level is reached). If that sufficiency level is not reached, the finetuning procedure can follow a step-by-step procedure where an incremental number of subjects with similar characteristics is used. This can give an estimation of how much more data with similar characteristics is required for reliable and sufficient performance. User of the system 600 can then weigh whether it is beneficial to search for additional available datasets or wait until more internal data on that particular population is gathered.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments of the present disclosure may be implemented on a computer system. The computer system may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system may comprise any circuit or combination of circuits. In one embodiment, the computer system may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), or any other type of processor or processing circuit. Other types of circuits that may be included in the computer system may be a custom circuit, an application-specific integrated circuit (ASlC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system may also include a display device, one or more speakers, and a keyboard and/or controller, which can include a mouse, trackball, touch screen, voice-recognition device, or any other device that permits a system user to input information into and receive information from the computer system.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the present disclosure can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the present disclosure comprise a data carrier having electronically readable control signals, which are capable of cooperating with a programmable computer system, such that one of the methods described herein is performed.

Generally, embodiments of the present disclosure can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present disclosure is, therefore, a computer program having a program code for performing one of the methods described herein, when the computer program runs on a computer.

A further embodiment of the present disclosure is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitory. A further embodiment of the present disclosure is an apparatus as described herein comprising a processor and the storage medium.

A further embodiment of the present disclosure is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the present disclosure comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

## Claims

1. A method (100) of training a sleep-related event classification model, wherein the method comprises:
providing (102) a pre-trained classification model, wherein the classification model is configured to classify one or more sleep-related events of a subject based on one or more physiological measurements of the subject, wherein the classification model is pre-trained using first training data associated with a source population of subjects; and
finetuning (104) the classification model using second training data associated with a target population of subjects.

2. The method of claim 1, further comprising:
determining an actual or expected insufficient performance of the pre-trained classification model for the subject;
wherein the step of finetuning the classification model is performed in response to the step of determining the insufficient performance.

3. The method of claim 2, wherein the step of determining the insufficient performance of the pre-trained classification model for the subject comprises:
determining that at least one characteristic of the subject mismatches the source population of subjects.

4. The method of any one of the preceding claims, further comprising:
selecting the second training data such that at least one characteristic of the subject which mismatches the source population of subjects matches the target population of subjects;
wherein the step of finetuning the classification model is performed using the selected second training data;
wherein the method comprises incrementally selecting portions of second training data and finetuning the classification model using the selected portion of second training data until a performance of the classification model is sufficient.

5. The method of claim 3 or 4, wherein the at least one characteristic of the subject comprises one or more of:
an age of the subject;
a medical disorder of the subject, in particular a sleep-related medical disorder, such as insomnia, obstructive sleep apnea, or rapid eye movement sleep behavior disorder;
a comorbid, non-sleep related, medical disorder;
a body mass index of the subject;
a medicine use of the subject;
a therapy use of the subject;
information about an environment of the subject.

6. The method of any one of the preceding claims, further comprising:
applying the pre-trained classification model to one or more physiological measurements of the subject to determine an amount of second training data for the step of finetuning.

7. A method (200) of classifying one or more sleep-related events of a subject, wherein the method comprises:
providing (202) a trained classification model trained in accordance with the method (100) of any one of claims 1-6;
providing (204) one or more physiological measurements of the subject;
classifying (206) one or more sleep-related events of the subject based at least in part on the one or more physiological measurements using the classification model to generate a classification result; and
providing (208) the classification result.

8. The method of any one of the preceding claims, wherein the classification model comprises a machine-learning model, more particularly a deep learning model, more particularly at least one neural network, more particularly at least one convolutional neural network, more particularly DeepSleepNet or TinySleepNet.

9. The method of any one of the preceding claims, wherein the one or more physiological measurements include one or more of: polysomnography data, electroencephalography data, electromyography data, photoplethysmography data, electrocardiography data, electrooculography data, actigraphy data, heart rate variability data, movement pattern data, breathing pattern data, and peripheral oxygen saturation data.

10. The method of any one of the preceding claims, wherein the classification model is configured to classify a sleep-related event of the subject into one or more of:
a waking stage;
a sleep stage, such as a non-rapid eye movement sleep stage or a rapid eye movement sleep stage, wherein the non-rapid eye movement sleep stage comprises a stage 1 non-rapid eye movement sleep stage, a stage 2 non-rapid eye movement sleep stage or a stage 3 non-rapid eye movement sleep stage;
a cortical event, such as an arousal, sleep spindle, a K-complex, a slow wave;
a non-cortical event, such as an obstructive or central apnea, a hypopnea, a respiratory effort related arousal.

11. The method of any one of the preceding claims, wherein the first training data associated with the source population maps one or more physiological measurements of subjects of the source population onto a plurality of sleep stages, in particular the plurality of sleep stages according to claim 10.

12. The method of any one of the preceding claims, wherein the sleep stage classification model has been pre-trained using supervised or semi-supervised learning.

13. A computer program, or a computer-readable medium storing a computer program, the computer program comprising instructions which, when the program is executed on a computer and/or a computer network, cause the computer and/or the computer network to carry out the method of any one of claims 1-12.

14. A data processing apparatus or system comprising means for carrying out the method of any one of claims 1-12.

15. A data structure comprising a trained sleep stage classification model trained using the method of any one of claims 1-6 or 8-12.
